# EUROPEAN PATENT APPLICATION

(11) **EP 4 803 201 A1**
(43) Date of publication of application: **09.09.2026**
(21) Application number: 23961541.2
(22) Date of filing: 15.12.2023
(51) Int. Cl.: B03C 1/00, G01N 33/543, G01N 33/552

(54) **MAGNETIC PARTICLE SEPARATION DEVICE**

(71) Applicant: Hitachi High-Tech Corporation, Minato-ku Tokyo 105-6409 (JP)
(72) Inventor: YAMAMOTO Shigeru, Tokyo 105-6409 (JP); FUJIOKA Michiru, Tokyo 105-6409 (JP); YABUHARA Tadao, Tokyo 105-6409 (JP)
(74) Representative: Strehl & Partner mbB
(86) International application number: PCT/JP2023/045107
(87) International publication number: WO 2025/126479

(57) **Abstract**

Provided is a magnetic particle separation device in which an adsorption amount of magnetic powder in a liquid in an end part can be made substantially equal to that in a center part even when using a sample container having the same size as a conventional one. The magnetic particle separation device includes: a plurality of sample containers in which a mixture composed of a liquid and magnetic particles is accommodated; a first magnet arranged for each of the plurality of sample containers and capable of moving up and down independently of the sample container; a magnetic body container that covers the first magnet; and a hole plate having holes into which the plurality of sample containers can be placed. In the hole plate, at least side surface parts of the holes close to the sample containers are made of a soft magnetic body.

## Description

### Technical Field

The present invention relates to a magnetic particle separation device that separates magnetic particles from a liquid in which the magnetic particles are suspended.

### Background Art

In recent years, information obtained by nucleic acid analysis such as cancer genomic inspection using a nextgeneration sequencing (NGS) system has been used in various fields such as medical care, clinical inspection, pharmaceutical industries, and food industries. In such nucleic acid analysis, nucleic acid extraction from various biological samples such as a blood, a tissue, and a cultured cell is an essential pretreatment.

As nucleic acid extraction methods, a method based on a property that a nucleic acid binds to silica in the presence of a chaotropic agent and a method based on a property that a nucleic acid binds to silica in the presence of an organic solvent are generally used instead of methods using phenol, chloroform, and the like, which are hazardous organic solvents. Using these methods, a nucleic acid extraction method using a nucleic acid capturing tip including a solid phase containing silica as a nucleic acid capturing carrier, and a method using magnetic beads (nucleic acid capturing carrier) whose surface is covered with silica have been reported. These methods include a step of binding the nucleic acid to the nucleic acid capturing carrier and an elution step of eluting the nucleic acid from the nucleic acid capturing carrier using an elution liquid.

In the method using the magnetic beads, after the elution step, the magnetic beads are recovered from the elution liquid using a magnet. PTL 1 discloses, as a method for efficiently collecting magnetic beads, a method of collecting magnetic beads on a wall of a container first and then recovering the magnetic beads using a magnetic rod. It is disclosed that, at this time, a difference in magnetic flux density distribution between an end portion and a center portion of a sample container is prevented by setting magnetization directions of adjacent magnets to different directions and magnetically binding the adjacent magnets.

### Citation List

### Patent Literature

PTL 1: JP2007-520331A

### Summary of Invention

### Technical Problem

In a DNA extraction and purification device in which a plurality of sample containers each accommodating a mixture of a liquid containing a DNA and surface-treated magnetic particles that adsorb the DNA are arranged vertically and horizontally, when a magnet container and a combination of a magnetic rod and a magnet independently move up and down with respect to the sample containers to perform a DNA adsorption reaction by mixing, and then "magnetism collection" of collecting the magnetic particles on a surface of the magnet container near the magnet is performed, magnetic binding between the magnets occurs in small containers in which a distance between the containers is short, and the magnetic flux density distribution in the liquid is different and an adsorption amount of a magnetic powder is different between the end portion and the center portion of the sample container.

In PTL 1, the difference in magnetic flux density distribution between the end portion and the center portion of the sample container is prevented by setting the magnetization directions of the adjacent magnets to different directions and magnetically binding the adjacent magnets, but the number of adjacent magnets in the end portion is small, and as a result, a difference occurs between the end portion and the center portion. The difference can be reduced by disposing magnets more than the number of the sample containers, but there is a problem that a space increases and the number of magnets also increases.

An object of the invention is to provide a magnetic particle separation device in which an adsorption amount of a magnetic powder in a liquid in an end portion can be made substantially equal to that in a center portion even when using a sample container having the same size as one in the related art.

### Solution to Problem

To achieve the above object, the invention has the following configuration.

A magnetic particle separation device includes: a plurality of sample containers each configured to accommodate a mixture containing a liquid and magnetic particles; first magnets disposed for corresponding one of the plurality of sample containers and capable of moving up and down independently of the sample container; a magnetic body container configured to cover the first magnets; and a hole plate having holes into which the plurality of sample containers can be placed, in which the hole plate contains a soft magnetic body in at least a side surface portion approaching the sample container.

### Advantageous Effects of Invention

It is possible to provide a magnetic particle separation device in which an adsorption amount of a magnetic powder in a liquid in an end portion can be made substantially equal to that in a center portion even when using a sample container having the same size as one in the related art.

### Brief Description of Drawings

[FIG. 1] FIG. 1 is a perspective view of a nucleic acid extraction device according to the invention.
[FIG. 2] FIG. 2 is a perspective cross-sectional view of the nucleic acid extraction device according to the invention.
[FIG. 3] FIG. 3 is a diagram showing an example of magnet body containers 4 having an array of 8 × 12.
[FIG. 4] FIG. 4 is a top view of a hole plate and holes.
[FIG. 5] FIG. 5 is a perspective cross-sectional view of the hole plate.
[FIG. 6] FIG. 6 is a diagram showing positions of magnetic flux density evaluation points.
[FIG. 7] FIG. 7 is a diagram showing magnetic flux density differences between an end portion and a center portion of the hole plate.
[FIG. 8] FIG. 8 is a diagram showing a state in which a position of the hole plate is changed.
[FIG. 9] FIG. 9 is a diagram showing calculation results of magnetic flux density distribution differences in FIG. 8.
[FIG. 10] FIG. 10 is a diagram showing positions of a hole plate upper end surface and a hole plate lower end surface.
[FIG. 11] FIG. 11 is a diagram showing calculation results of magnetic flux density distribution differences in FIG. 10.
[FIG. 12] FIG. 12 is a diagram showing a state in which a position of an upper magnet is changed.
[FIG. 13] FIG. 13 is a diagram showing an embodiment using the upper magnet and a lower magnet.
[FIG. 14] FIG. 14 is a diagram showing a magnetism collection flow of magnetic particles using the upper magnet and the lower magnet.
[FIG. 15] FIG. 15 is a diagram showing the positions of the hole plate upper end surface and the hole plate lower end surface.
[FIG. 16] FIG. 16 is a diagram showing calculation results of magnetic flux density distribution differences in FIG. 15.
[FIG. 17] FIG. 17 a diagram showing an embodiment in which tapered holes are provided.
[FIG. 18] FIG. 18 a diagram showing an embodiment in which grooves are provided.
[FIG. 19] FIG. 19 is a diagram showing a difference in drive force depending on the presence or absence of tapering.

### Description of Embodiments

Hereinafter, embodiments of the invention will be described with reference to the drawings.

### Embodiment 1

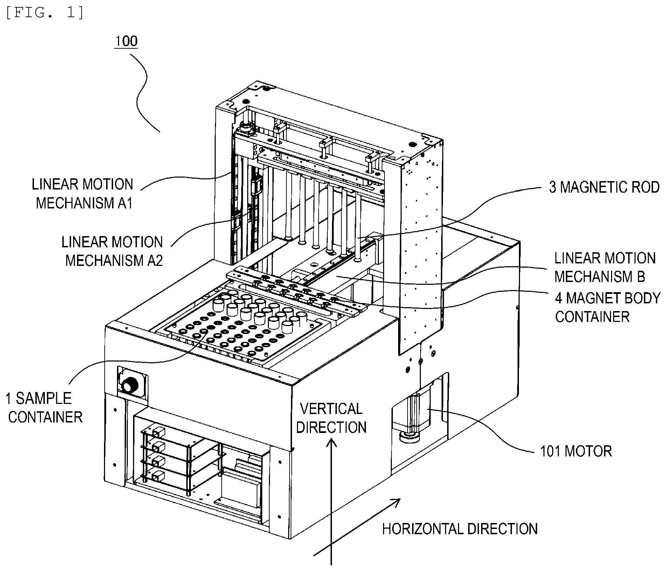

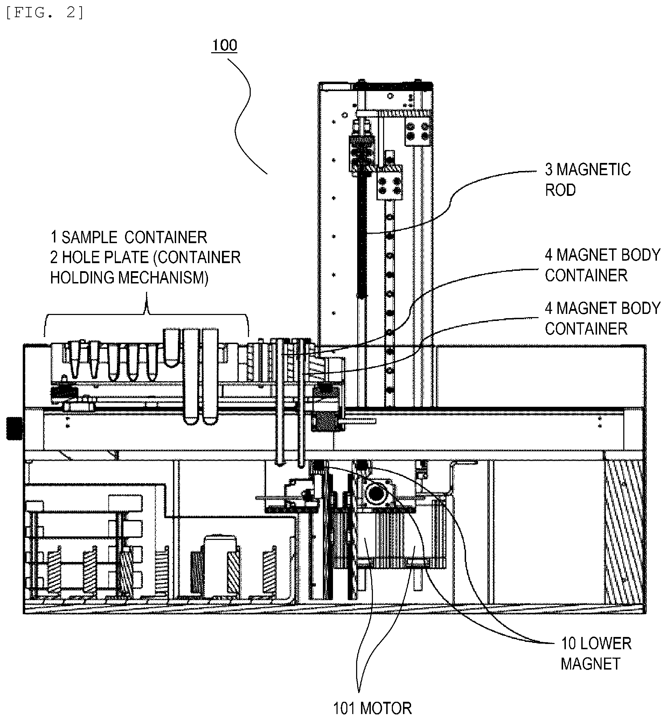

FIGS. 1 and 2 are a perspective view and a perspective cross-sectional view of a nucleic acid extraction device 100 according to the invention, respectively. The nucleic acid extraction device 100 is a device for extracting a nucleic acid from a sample containing the nucleic acid and magnetic particles.

The nucleic acid extraction device 100 includes a linear motion mechanism A1 that moves a magnetic rod 3 in a vertical direction, a linear motion mechanism A2 that moves a magnet body container 4 in the vertical direction, a linear motion mechanism B that moves a sample container 1 in a horizontal direction together with a hole plate (container holding member) 2 that holds the sample container 1, and a motor 101 that drives these linear motion mechanisms. The sample container 1 is held by the hole plate (container holding member) 2 to be described later.

The linear motion mechanism B moves the magnetic body container 4 (also referred to as magnet body container) below the magnetic rod 3, and the linear motion mechanism A1 moves the magnetic rod 3 up and down, so that the magnet body container 4 can be attached to and detached from the magnetic rod 3. Further, the linear motion mechanism B moves the sample container 1 below the magnetic rod 3 (to which the magnet body container 4 is attached) and moves the magnet body container 4 (and the magnetic rod 3) up and down, so that a liquid in the sample container 1 can be mixed by the magnet body container 4.

In this figure, the magnet body containers 4 have different thicknesses, but may have the same thickness.

Although the nucleic acid extraction device 100 shown in FIG. 2 includes a lower magnet 10, the lower magnet 10 is used in [Embodiment 2] and is not used in [Embodiment 1]. In [Embodiment 2], the lower magnet 10 will be described again.

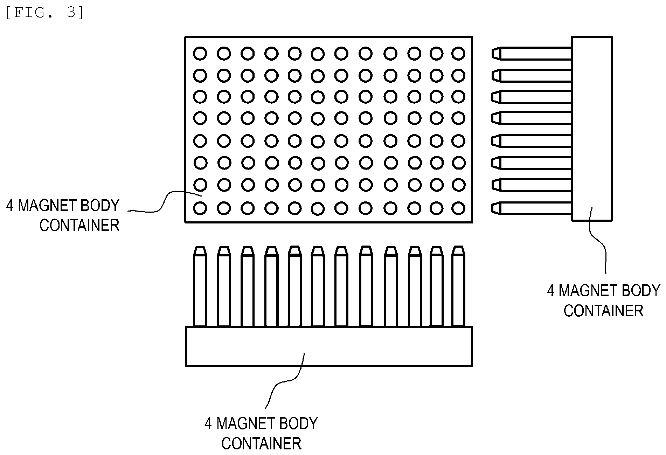

In the present embodiment, six magnetic rods 3 are arranged in a horizontal row, but a device including a plurality of rows of magnetic rods 3 may be used. For example, eight rows of magnetic rods 3 may be provided, and the magnetic rods 3 of 6 × 8 = 48 may be provided. With this configuration, the magnetic rods 3 can be simultaneously inserted into all the sample containers 1 placed in the hole plate 2, and magnetic particles can be simultaneously collected in all the sample containers 1. In this case, the magnet body containers 4 are also formed in a matrix manner as the magnetic rods 3. FIG. 3 is a diagram showing an example of the magnet body containers 4 having an array of 8 × 12.

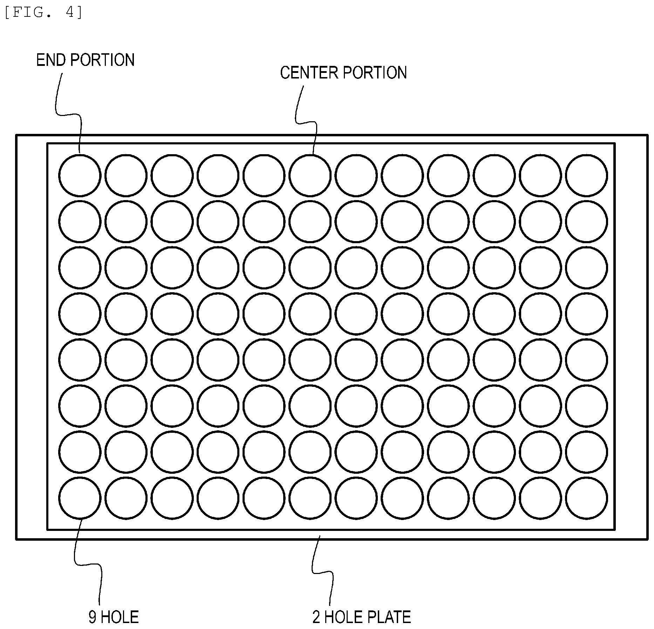

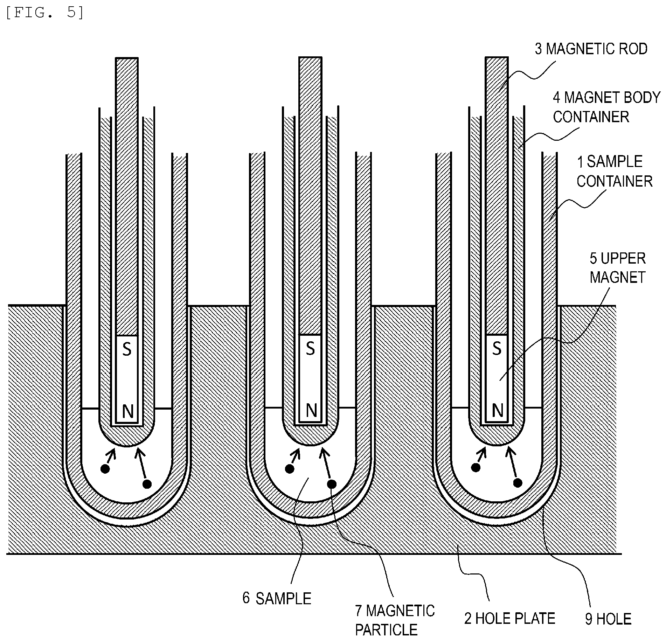

Next, the hole plate will be described in detail. FIG. 4 shows the hole plate 2 having holes 9 in which eight sample containers 1 can be arranged vertically and twelve sample containers 1 can be arranged horizontally. FIG. 5 is a perspective cross-sectional view of the hole plate 2. By inserting the sample containers 1 into the holes 9 of the hole plate 2, the sample containers 1 can be arranged. In order to collect only magnetic particles 7 from a sample 6 in which the magnetic particles 7 are suspended in the sample container 1, the magnetic rod 3, an upper magnet 5 bound to the magnetic rod 3, and the magnet body container 4 (also referred to as a magnetic body cover) covering the magnetic rod 3 and the upper magnet 5 are inserted into the sample container 1, the magnetic body container 4 is moved up and down to cause a DNA adsorption reaction by mixing, and then "magnetism collection" is performed to collect the magnetic particles 7 on a surface of the magnet body container 4 near the upper magnet 5. In FIG. 5, magnetization directions of the upper magnets 5 are all the same (disposed such that N poles are located on a lower side in the figure).

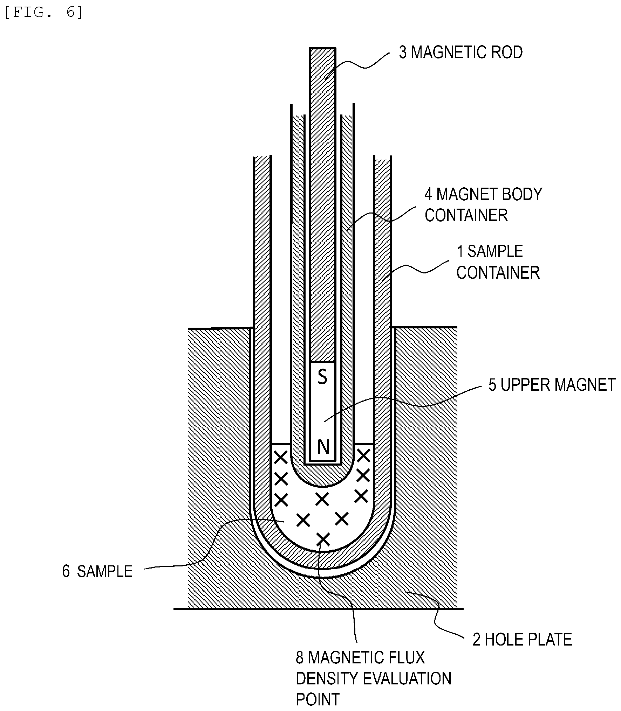

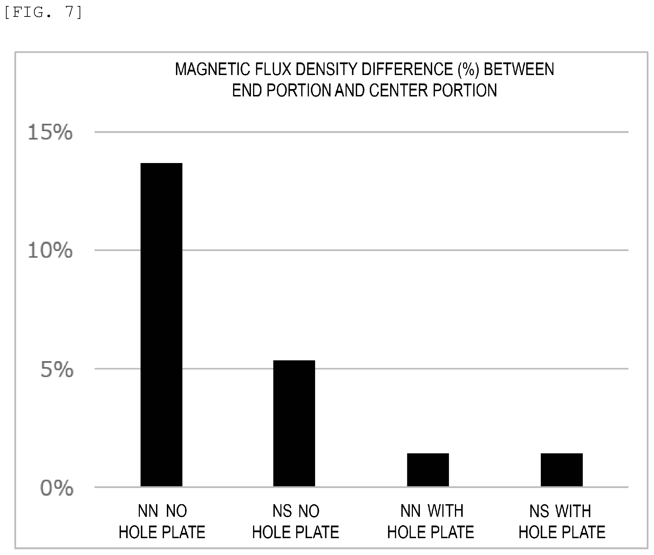

FIG. 7 shows results of calculating a magnetic flux density at magnetic flux density evaluation points 8 (each point indicated by X in FIG. 6) in a liquid in which the magnetic particles 7 are present by magnetic field analysis, and comparing a difference in magnetic flux density between an end portion and a center portion of the hole plate 2 in FIG. 4. A difference of 14% is observed in "NN no hole plate" without the hole plate 2 made of a soft magnetic body, in which the magnetization directions of the upper magnets 5 are the same direction (NN means that the magnets are arranged with the N pole and the N pole at the same end), and it is possible to reduce the difference to 5% by adopting "NS no hole plate" in which magnetization directions described in PTL 1 are arranged to face each other (NS means that the magnets are arranged with the N pole, a S pole, and the N pole... at the same end). It can be seen that the use of "NN with hole plate" or "NS with hole plate" of the invention can further reduce the difference to 1%.

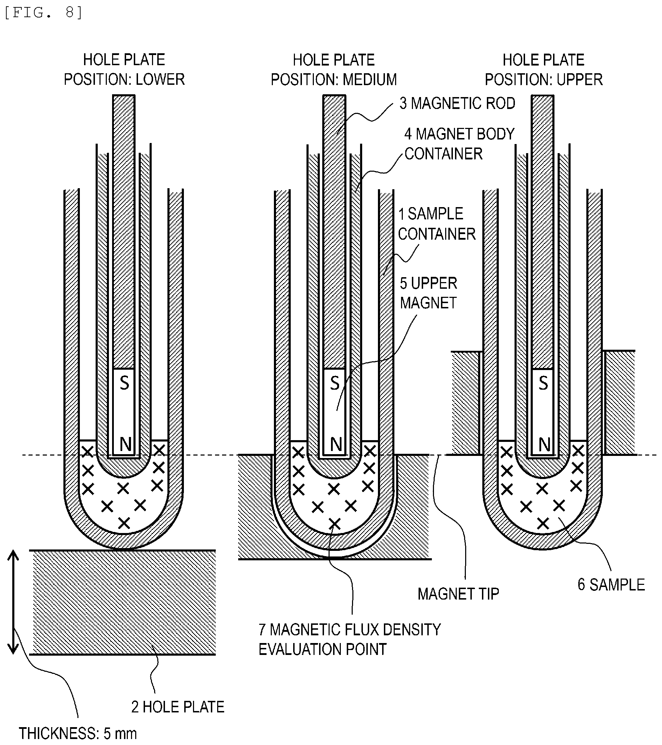

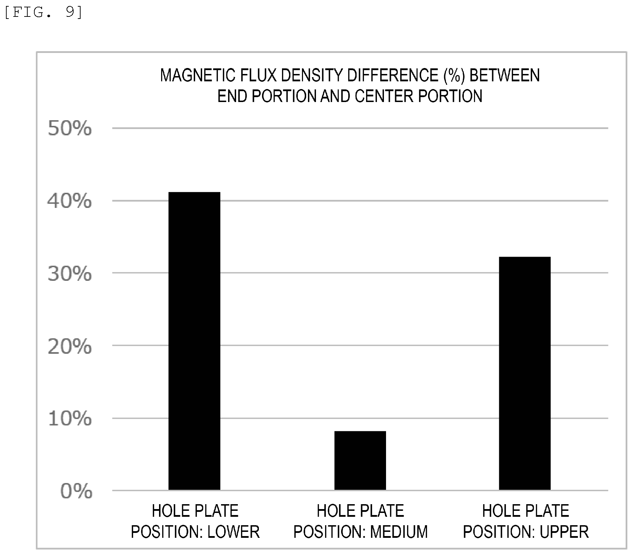

During the magnetism collection, the upper magnet 5 and the magnet body container 4 operate synchronously in the vertical direction to collect the magnetic particles 7. The hole plate 2 (also referred to as a sample container holder) has holes 9 for accommodating sample containers 1. It is ideal that the hole 9 covers all of the upper magnet 5, the sample 6, and the magnetic rod 3, but the sample container 1 generally has a hemispherical bottom surface and a cylindrical upper portion, and it is difficult in design to cover the upper portion of the sample container 1 with a hole plate. FIG. 9 shows a difference in magnetic flux density between the end portion and the center portion when a hole plate having a thickness of 5 mm is disposed as lower, medium, and upper positions as hole plate positions with respect to the sample container 1 as shown in FIG. 8. It can be seen from FIG. 9 that an end surface of the hole does not need to cover the entire upper magnet 5 and magnetic rod 3, it is most effective to cover a distance of about 5 mm downward from a magnet tip shown in FIG. 8, and even if the hole plate is not disposed at the upper portion of the container, the difference in magnetic flux density is not necessarily affected.

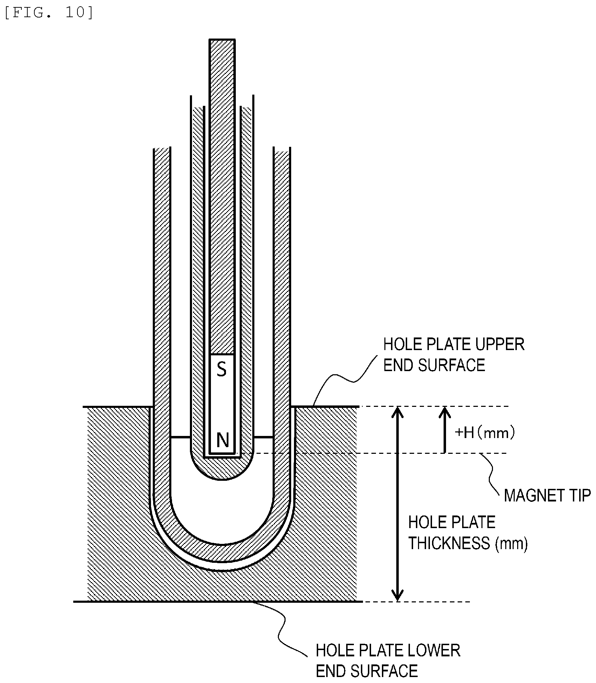

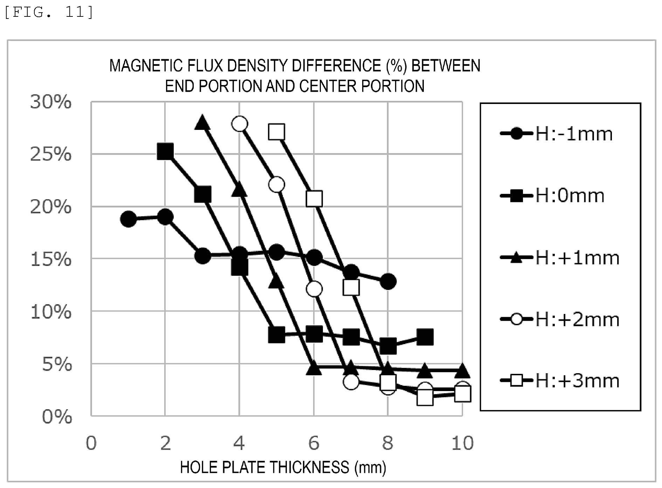

Further, FIG. 11 shows a difference in magnetic flux density between the end portion and the center portion when a thickness H from the magnet tip to a hole plate upper end surface is changed in units of 1 mm as shown in FIG. 10. From this, it can be seen that the hole plate upper end surface is desirably at a position about 2 mm higher than a magnet tip surface and desirably has a thickness of 7 mm or more up to a hole plate lower end.

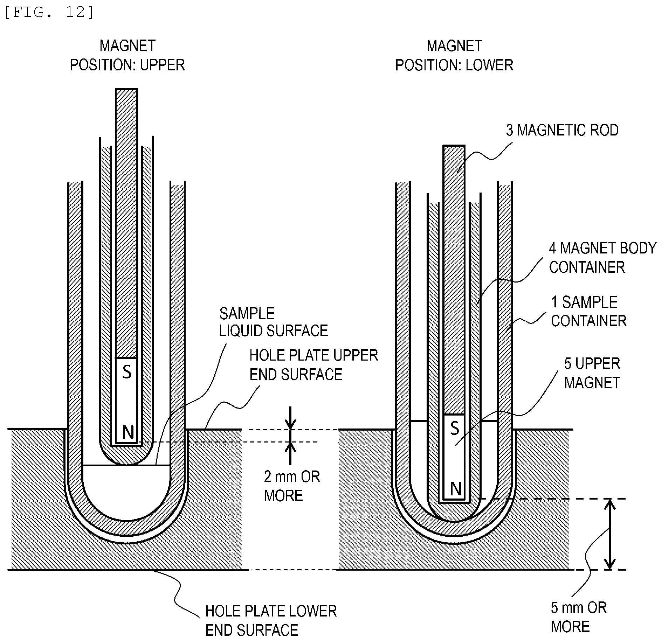

Next, FIG. 12 shows a relationship between a sample liquid surface and a magnet position. Considering that the magnetic body container 4 and the upper magnet 5 can move independently of the sample container 1, and the adsorption of a magnetic powder mainly occurs from a state of "magnet position: upper" in which a tip of the magnet body container 4 descends and comes into contact with the sample liquid surface, it can be seen that, in the case of the dimensional design shown in this figure, the hole plate upper end surface is desirably higher than the magnet tip surface by 2 mm or more when the magnet body container 4 comes into contact with the sample liquid surface, and is desirably 5 mm or more from the magnet tip surface in a state of "magnet position: lower" when the magnet body container 4 is attached to the bottom surface of the sample container 1.

Since inner and outer diameters of the sample container 1, a size of the upper magnet 5, and an amount of the sample 6 are different for each device, and a magnetic binding state between the upper magnet 5 and the hole plate 2 is also different, it is needless to say that ideal dimension values are different for each device.

The hole plate 2 may have a lattice structure in which plates subjected to sheet metal processing are combined, in addition to a perforated soft magnetic body. When a metal soft magnetic body is used, the soft magnetic body may also have a temperature adjustment function due to excellent thermal conductivity thereof. A hole may be formed in a non-magnetic material such as a resin, and a cylindrical or container-shaped soft magnetic body may be inserted thereto. The hole plate 2 may have a structure that receives only one sample container 1 since the hole plate 2 has a magnetic force strengthening effect and a magnetic shielding effect. The soft magnetic body includes a permalloy, pure iron, an iron-based alloy, a nickel alloy, a cobalt alloy, ferrite, a resin material containing a magnetic powder, and the like.

### Embodiment 2

In the present embodiment, the lower magnet 10 (also referred to as a bottom magnet) is further provided. FIG. 2 is used for the description again. The nucleic acid extraction device 100 includes the lower magnet 10. The lower magnet 10 is disposed so as to be located below the sample container 1 when the sample container 1 moves below the magnetic rod 3. The lower magnet 10 can be moved up and down by, for example, the linear motion mechanism A2. When the lower magnet 10 approaches a bottom surface of the sample container 1, magnetic beads can be collected at the bottom surface of the sample container 1. A plurality of types of lower magnets 10 may be provided and selected, such as one used for a large sample container 1 and one used for a small sample container 1, or only one lower magnet 10 may be used.

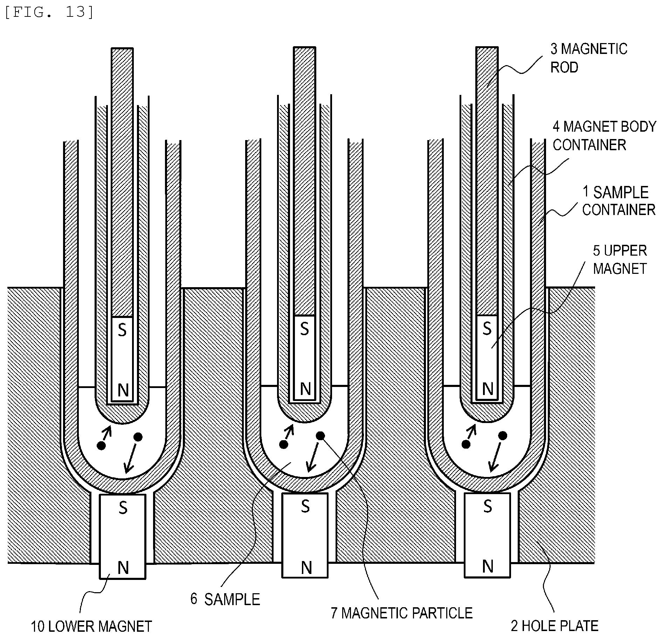

FIG. 13 is a perspective cross-sectional view of a hole plate when the lower magnet 10 is used. The lower magnet 10 can be vertically driven independently of the upper magnet 5. The tip of the magnet body container 4 has a hemispherical surface for promoting mixing of the sample 6 and the magnetic particles 7, and is thick in consideration of ease of molding, and there is a disadvantage that a distance between the upper magnet 5 and the surface of the magnetic body container 4 increases and an adsorption force decreases. On the other hand, when the lower magnet 10 is disposed at a bottom of the thin magnet body container 4, there is an advantage that the adsorption force is high and a magnetism collection time can be shortened.

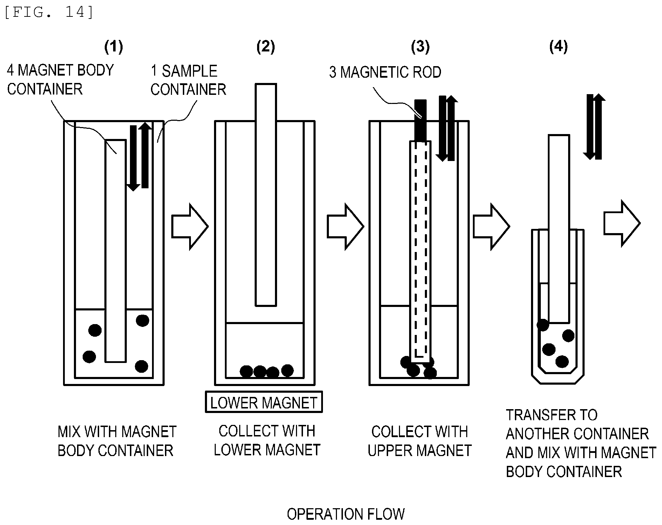

A magnetism collection flow of the magnetic particles 7 using the upper magnet 5 and the lower magnet 10 will be described with reference to FIG. 14.

First, only the magnetic body container 4 is immersed in a liquid in which the magnetic particles 7 are suspended in the sample container 1, and the liquid is mixed by moving the magnetic body container 4 up and down (1). Next, the magnetic body container 4 is pulled up to be above the liquid surface, then the lower magnet 10 is moved upward, and the lower magnet 10 is brought close to the bottom of the sample container 1. Accordingly, the magnetic particles 7 suspended in the liquid can be collected at one place near the lower magnet 10 (2). Next, the upper magnet 5 is immersed in the liquid simultaneously with the magnet body container 4, and the magnetic particles 7 collected at one place near the lower magnet 10 are collectively collected (3). During the magnetism collection, the magnet body container 4 may be moved up and down in the liquid. The magnet body container 4 is immersed in a liquid in another container together with the upper magnet 5, and then the upper magnet 5 is moved upward. By moving only the magnet body container 4 up and down in the liquid in another container, the magnetic particles 7 collected on the surface of the magnet body container 4 are suspended in the liquid in another container (4).

In Embodiment 1 which is an embodiment in which the lower magnet 10 is not used, the magnetism collection is performed only by the upper magnet. In contrast, in Embodiment 2, since the magnetic particles 7 can be once collected at one place using the lower magnet 10 and the collected magnetic particles 7 can be directly collected using the upper magnet 5, the time required for the magnetism collection is shorter than that in Embodiment 1, that is, a throughput is higher. On the other hand, in Embodiment 2, since a mechanism for moving the lower magnet 10 up and down and the lower magnet 10 are used, the cost is increased as compared with the device according to Embodiment 1, and the device may be slightly enlarged. It is desirable to appropriately select to use only the upper magnet 5 or to use the lower magnet 10 in combination according to magnetism collection performance required for the device.

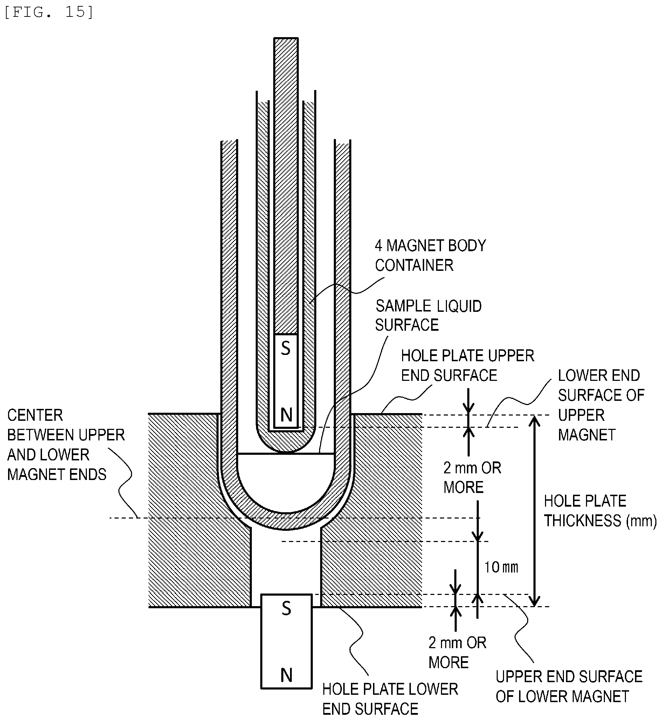

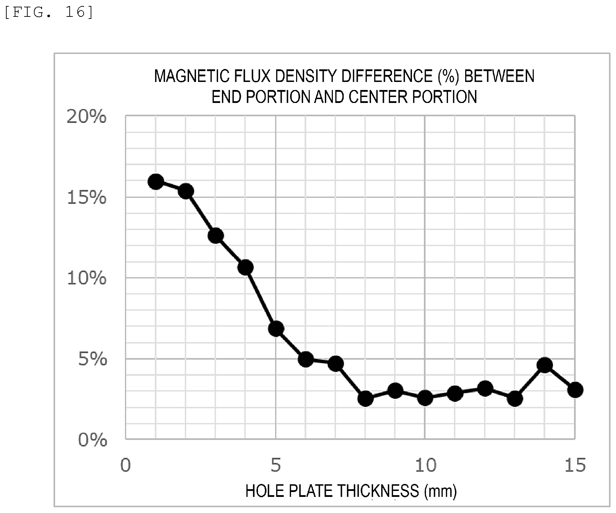

Next, an optimum value of the thickness of the hole plate when the lower magnet 10 is used will be described with reference to FIG. 15. As shown in FIG. 15, when it is considered that the adsorption force is generated mainly when the tip of the magnet body container 4 containing the upper magnet 5 comes into contact with the liquid surface of the sample 6 or the lower magnet 10 approaches 10 mm from the bottom of the sample 6, it is desirable that a hole plate upper end surface is located, by 2 mm or more, above a tip surface (lower end surface) of the upper magnet 5 when the tip of the magnet body container 4 comes into contact with the liquid surface of the sample 6, and a hole plate lower end surface is located, by 2 mm or more, below a position when the lower magnet 10 is located 10 mm from the bottom of the sample 6. FIG. 16 shows an analysis result of a difference in magnetic flux density between the end portion and the center portion when a distance between the end surfaces of the upper and lower magnets is 5 mm and a thickness center is provided at a center between the upper and lower magnet ends. It can be seen that the difference in magnetic flux density is minimized when the thickness is 9 mm or more, and an overlap of 2 mm or more may be provided for each magnet.

### Embodiment 3

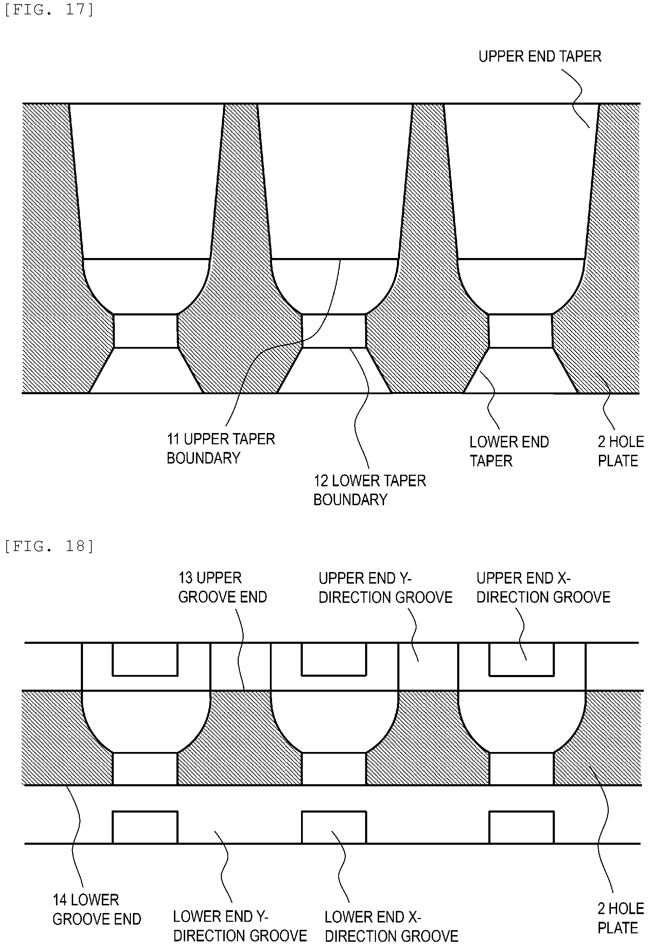

When the hole plate 2 made of a soft magnetic body is used as in Embodiments 1 and 2 and a distance between the hole plate 2 and the magnet is a certain distance once the magnet is brought close to or separated from the hole plate 2, there is a problem that a magnetic force rapidly increases and a load on a drive source (motor or the like) for driving the magnetic rod 3 or the like increases. As a countermeasure, as shown in FIGS. 17 and 18, tapered holes or grooves having different depths may be provided at the (bottom) end portion of the hole plate 2 such that the magnetic force between the soft magnetic body and the magnet gradually decreases. A total load may be reduced by making a taper angle or a shape different for each hole and shifting a peak position of the load (shifting a phase).

A portion between an upper taper boundary 11 and a lower taper boundary 12 in FIG. 17 is narrower than the sample container 1, corresponds to the hole plate upper end surface and the hole plate lower end surface shown in Embodiments 1 and 2, and is a range in which the effect of minimizing the difference in magnetic flux density between the end portion and the center portion is obtained. Similarly, a portion between an upper groove end 13 and a lower groove end 14 in FIG. 18 corresponds to the hole plate upper end surface and the hole plate lower end surface in Embodiments 1 and 2, and is a range in which the effect of minimizing the difference in magnetic flux density between the end portion and the center portion is obtained.

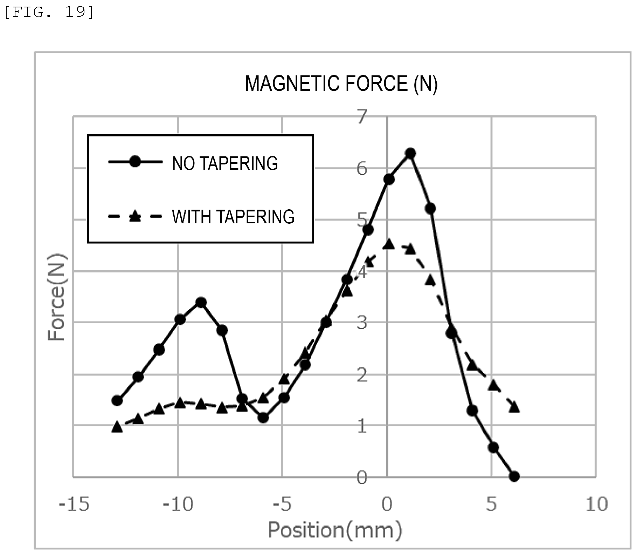

FIG. 19 shows a magnetic force generated between the magnet and the hole plate 2 at each position. It can be seen that a peak position of the magnetic force is smaller by tapering as compared with a case where the tapering is not performed, and a total (energy) of the force necessary for driving, which is indicated by an integral value of a graph of FIG. 19, is also reduced.

### Reference Signs List

1: sample container
2: hole plate
3: magnetic rod
4: magnet body container
5: upper magnet
6: sample
7: magnetic particle
9: hole
10: lower magnet

## Claims

1. A magnetic particle separation device comprising:
a plurality of sample containers each configured to accommodate a mixture containing a liquid and magnetic particles;
first magnets disposed for corresponding one of the plurality of sample containers and capable of moving up and down independently of the corresponding sample container;
a magnetic body container configured to cover the first magnets; and
a hole plate having holes into which the plurality of sample containers are capable of being placed, wherein
the hole plate contains a soft magnetic body in at least a side surface portion approaching the sample container.

2. The magnetic particle separation device according to claim 1, wherein
the soft magnetic body forms a magnetic path between the plurality of sample containers.

3. The magnetic particle separation device according to claim 1, wherein
the plurality of first magnets are disposed so as to have magnetic poles in a same direction.

4. The magnetic particle separation device according to claim 3, further comprising:
a second magnet capable of being disposed at a bottom of the hole into which the plurality of sample containers are capable of being placed, wherein
a magnetic pole of the second magnet is disposed in a direction same as the direction of the magnetic poles of the first magnets.

5. The magnetic particle separation device according to any one of claims 1 to 4, wherein
the holes each have a taper in which a hole diameter increases toward an opening or have grooves having different depths.
